# EUROPEAN PATENT APPLICATION

(11) **EP 4 674 952 A1**
(43) Date of publication of application: **07.01.2026**
(21) Application number: 24779681.6
(22) Date of filing: 18.03.2024
(51) Int. Cl.: C12N 5/071, C12N 5/077

(54) **THREE-DIMENSIONAL MAMMARY GLAND MODEL AND METHOD FOR PRODUCING SAME**

(30) Priority: 30.03.2023 JP 2023055898
(71) Applicant: Toppan Holdings Inc., Tokyo 110-0016 (JP); The University of Osaka, Suita-shi Osaka 565-0871 (JP)
(72) Inventor: SUZUKI, Mizuho, Tokyo 110-0016 (JP); MATSUSAKI, Michiya, Suita-shi, Osaka 565-0871 (JP); LOUIS, Fiona, Suita-shi, Osaka 565-0871 (JP)
(74) Representative: Hasegawa, Kan
(86) International application number: PCT/JP2024/010604
(87) International publication number: WO 2024/203556

(57) **Abstract**

A three-dimensional mammary gland model includes a three-dimensional tissue containing cells and fragmented extracellular matrix components, and the cells include mature adipocytes and mammary epithelial cells.

## Description

### Technical Field

The present invention relates to a three-dimensional mammary gland model and a method for producing the same.

### Background Art

Various studies have been conducted so far on the preparation of mammary gland models (Non-Patent Literature 1 and 2 and Patent Literature 1). Non-Patent Literature 1 discloses a mammary gland model in which fibroblasts and mammary epithelial cells are embedded in Matrigel. Non-Patent Literature 2 discloses a mammary gland model using primary mouse mammary gland organoids cultured in Matrigel. Patent Literature 1 discloses a cell culture method in which mammary epithelial cells are cultured in a stratified state in a partitioned microspace to obtain a tissue structure with biomimetic functions.

### Citation List

### Patent Literature

[Patent Literature 1] PCT International Publication No. WO2009/099153

### Non-Patent Literature

[Non-Patent Literature 1] Zuzana Koledova ed., "3D Cell Culture: Methods and Protocols", Methods in Molecular Biology, vol. 1612.
[Non-Patent Literature 2] Sumbal J, et al., "Primary Mammary Organoid Model of Lactation and Involution.", Front Cell Dev Biol. 2020Mar 19;8:68.

### Summary of Invention

### Technical Problem

However, it is not possible to obtain milk from the mammary gland tissue model disclosed in Non-Patent Literature 1 or from a tissue structure obtained using the mammary epithelial cells disclosed in Patent Literature 1. Furthermore, a method for producing the tissue structure disclosed in Patent Literature 1 needs to use a special cell culture vessel, which is not convenient. Casein, one of milk proteins, has been detected in the mammary gland model disclosed in Non-Patent Literature 2, but this could not be reproduced using cells other than primary mouse mammary gland organoids. As such, it has been difficult to prepare a mammary gland model in which mammary epithelial cells have been differentiated to a point where milk can be obtained in vitro.

An object of the present invention is to provide a three-dimensional mammary gland model from which milk can be obtained. Further, an object of the present invention is to provide a method for easily producing the above-mentioned three-dimensional mammary gland model.

### Solution to Problem

That is, the present invention includes, for example, the following inventions.
[1] A three-dimensional mammary gland model consisting of a three-dimensional tissue, comprising cells and fragmented extracellular matrix components,
   in which the cells comprise mature adipocytes and mammary epithelial cells.
[2] The three-dimensional mammary gland model according to [1], having a void surrounded by two layers of mammary epithelial cells.
[3] The three-dimensional mammary gland model according to [1] or [2], in which, in the two layers of mammary epithelial cells, the mammary epithelial cells on a luminal side are CK8/18 positive cells, and the mammary epithelial cells on the basement membrane side are CK14 positive cells.
[4] The three-dimensional mammary gland model according to any one of [1] to [3], in which the fragmented extracellular matrix components comprise fragmented collagen.
[5] The three-dimensional mammary gland model according to any one of [1] to [4], in which the three-dimensional tissue further comprises a hydrogel.
[6] The three-dimensional mammary gland model according to [5], in which the hydrogel is a fibrin gel.
[7] A method for producing a three-dimensional mammary gland model, comprising:
   a step of bringing cells into contact with fragmented extracellular matrix components in an aqueous medium; and
   a step of culturing the cells in contact with the fragmented extracellular matrix components,
      in which the cells comprise mature adipocytes, adipose-derived stem cells, and mammary epithelial cells.
[8] The method for producing the three-dimensional mammary gland model according to [7], in which the fragmented extracellular matrix components comprise fragmented collagen.
[9] The method for producing the three-dimensional mammary gland model according to [7] or [8], in which
   the contact step comprises further bringing a hydrogel precursor into contact with the fragmented extracellular matrix components, and
   the method further comprises a step of gelling the hydrogel precursor after the contact step and before the culturing step.
[10] The method for producing the three-dimensional mammary gland model according to [9], in which the hydrogel is a fibrin gel.

### Advantageous Effects of Invention

According to the present invention, it is possible to provide a three-dimensional mammary gland model from which milk can be obtained. According to the present invention, it is also possible to provide a method for easily producing the above-mentioned three-dimensional mammary gland model.

### Brief Description of Drawing

FIG. 1 is a schematic diagram showing a void surrounded by two layers of mammary epithelial cells.
FIG. 2 is an image (A) showing results of CK8/18 immunostaining of a three-dimensional tissue section in Test Example 1, an enlarged image (B) of a portion of FIG. 2(A), an image (C) showing results of CK14 immunostaining, and an enlarged image (D) of a portion of FIG. 2(C).
FIG. 3 is an image showing results of Nile red staining and CK8/18 immunostaining of a three-dimensional tissue in Test Example 1.
FIG. 4 is an image (A) showing results of Nile red staining of a three-dimensional tissue in Example 2 in Test Example 2, and an image (B) showing results of Nile red staining of a three-dimensional tissue in Comparative Example 1 in Test Example 2.
FIG. 5 is a graph comparing the fluorescence intensity of Nile red staining of a three-dimensional tissue in Test Example 2.
FIG. 6 is an image (A) showing results of Western blotting analysis of casein in a three-dimensional tissue in Test Example 3, and an image (B) showing results of Western blotting analysis of casein in two-dimensionally cultured mammary epithelial cells in Test Example 3.
FIG. 7 is a graph showing results of quantifying a band brightness of casein in a three-dimensional tissue and in two-dimensionally cultured mammary epithelial cells detected by Western blotting in Test Example 3.
FIG. 8 is a graph showing results of analyzing expression of CLDN3 in a three-dimensional tissue in Test Example 4.
FIG. 9 is a diagram showing an overview of an example of a mechanism leading to milk secretion in a mammary gland.

### Description of Embodiment

Hereinafter, embodiments of the present invention will be described in detail. However, the present invention is not limited to the following embodiment.

### [Three-dimensional mammary gland model]

A three-dimensional mammary gland model according to this embodiment is configured with a three-dimensional tissue containing cells and fragmented extracellular matrix components. The cells contain mature adipocytes and mammary epithelial cells. The three-dimensional mammary gland model according to this embodiment promotes differentiation of mammary epithelial cells and is capable of obtaining milk.

The three-dimensional mammary gland model according to this embodiment is a mammary gland model that can be artificially formed in vitro, and does not include mammary gland tissue itself that has merely been isolated from a living organism. Here, the mammary gland tissue in a living organism has a more complex structure than a three-dimensional tissue. For this reason, the three-dimensional mammary gland model according to this embodiment can be easily distinguished from a three-dimensional tissue and mammary gland tissue in a living organism.

In this specification, the term "three-dimensional tissue" refers to an assembly of cells (agglomerated cell mass) in which cells are disposed three-dimensionally via extracellular matrix components and/or fragmented extracellular matrix components, and is an assembly artificially created by cell culture. The shape of the three-dimensional tissue is not particularly limited, and examples thereof include a sheet shape, a spherical shape, a substantially spherical shape, an ellipsoidal shape, a substantially ellipsoidal shape, a hemispherical shape, a substantially hemispherical shape, a semicircular shape, a substantially semicircular shape, a rectangular shape, a substantially rectangular shape, and the like. The shape of the three-dimensional tissue is preferably a spherical shape or a substantially spherical shape, from the viewpoint of being closer to the shape of the mammary gland of a living organism.

The total number of cells configuring the three-dimensional tissue according to this embodiment is not particularly limited, and is determined appropriately in consideration of the thickness and shape of the three-dimensional mammary gland model to be constructed, the size of a cell culture vessel used for the construction, and the like. The total number of cells configuring the three-dimensional tissue according to this embodiment is also synonymous with the total number of cells configuring the three-dimensional mammary gland model according to this embodiment.

In this specification, "cells" are not particularly limited, and cells derived from mammals such as humans, monkeys, dogs, cats, rabbits, pigs, cows, mice, and rats may be used. The parts of cells derived are not particularly limited, and may be somatic cells derived from bones, muscles, internal organs, nerves, brain, bones, skin, blood, and the like or may be germ cells. In addition, the cells may be stem cells or may be cultured cells such as primary cultured cells, sub-cultured cells and cell line cells.

In this specification, the "stem cells" refers to cells having a self-replication ability and pluripotency. Stem cells include pluripotent stem cells having an ability to differentiate into any cell tumor and tissue stem cells (also referred to as somatic stem cells) having an ability to differentiate into specific cell tumors. Examples of pluripotent stem cells include embryonic stem cells (ES cells), somatic cell-derived ES cells (ntES cells) and induced pluripotent stem cells (iPS cells). Examples of tissue stem cells include mesenchymal stem cells (for example, adipose-derived stem cells and bone marrow-derived stem cells), hematopoietic stem cells and neural stem cells. Examples of adipose-derived stem cells (ADSCs) include human adipose-derived stem cells and bovine adipose-derived stem cells.

The three-dimensional tissue according to this embodiment contains at least mature adipocytes and mammary epithelial cells as cells.

Mature adipocytes are cells that fall within the concept of adipocytes, and can be determined, for example, using the size of lipid droplets as an indicator. Lipid droplets are cell organelles that store lipids such as triglycerides (neutral fats) and cholesterol, and the lipids are covered with a single layer of phospholipids to have a droplet-like shape. In addition, expression of adipose tissue-specific proteins (perilipin, and the like) on the surface of the phospholipids is observed. The size of the lipid droplets in mature adipocytes varies, but for example, when an average value of the size of a lipid droplet is 20 µm or more, the adipocytes can be considered to be mature adipocytes.

The mature adipocytes may be, for example, cells collected from subcutaneous adipose tissue, epicardial-derived adipose tissue, and the like, may be cells obtained by inducing differentiation of collected cells, or may be cells artificially differentiated from stem cells. The mature adipocytes are not particularly limited, but since the three-dimensional tissue according to this embodiment is used as a mammary gland model, it is preferable to use those derived from mammary gland adipose tissue.

The content of the mature adipocytes relative to the total number of cells in the three-dimensional tissue may be, for example, 5% or more, 10% or more, 15% or more, 20% or more, 25% or more, or 30% or more, or may be 95% or less, 90% or less, 80% or less, or 75% or less. Furthermore, from the viewpoint of being more suitable as a mammary gland model, the content of the mature adipocytes relative to the total number of cells in the three-dimensional tissue according to this embodiment may be 30% or more and 80% or less, 35% or more and 75% or less, or 40% or more and 65% or less.

The cells in the three-dimensional tissue according to this embodiment may contain undifferentiated adipocytes such as adipose-derived stem cells. The content of the adipose-derived stem cells may be, for example, 1% or more, 3% or more, 5% or more, 10% or more, or 15% or more, or 40% or less, 30% or less, 20% or less, 10% or less, or 5% or less, relative to the total number of cells in the three-dimensional tissue.

Mammary epithelial cells are cells that are present in the mammary gland and have functions such as synthesizing and secreting milk. Furthermore, mammary epithelial cells accumulate lipid droplets as differentiation progresses. Mammary epithelial cells that have accumulated lipid droplets have progressed to a state of differentiation where they can secrete milk. Thus, the mammary epithelial cells in the three-dimensional tissue according to this embodiment may be mammary epithelial cells that have accumulated lipid droplets.

The mammary epithelial cells may be, for example, primary mammary epithelial cells collected from the mammary gland of an animal, cultured primary mammary epithelial cells, a cultured cell line established from primary mammary epithelial cells, or mammary epithelial cells artificially differentiated from stem cells.

The content of the mammary epithelial cells relative to the total number of cells in the three-dimensional tissue may be, for example, 5% or more, 10% or more, 15% or more, 20% or more, 25% or more, 30% or more, 35% or more, 40% or more, 45% or more, or 50% or more, or may be 95% or less, 90% or less, 80% or less, 70% or less, 60% or less, 50% or less, 40% or less, or 30% or less. The content of the mammary epithelial cells relative to the total number of cells in the three-dimensional tissue according to this embodiment may be 15% or more and 70% or less, 15% or more and 35% or less, or 20% or more and 30% or less, from the viewpoint of being more suitable as a mammary gland model and from the viewpoint of being more likely to form a void surrounded by two layers of mammary epithelial cells to be described later.

In the three-dimensional tissue according to this embodiment, a ratio (cell number) of mature adipocytes to mammary epithelial cells is not particularly limited, but may be, for example, 0.25:1 to 2.5:1, or may be 1:1 to 2.4:1.

The cells may further include cells other than the mature adipocytes and the mammary epithelial cells. Examples of the cells other than the mature adipocytes and the mammary epithelial cells include fibroblasts (for example, human mammary fibroblasts (HMF), human skin-derived fibroblasts (NHDF), human cardiac fibroblasts (NHCF), human gingival fibroblasts (HGF), and the like), adipocytes other than mature adipocytes and adipose-derived stem cells, vascular endothelial cells (for example, human umbilical vein-derived vascular endothelial cells (HUVEC)), cancer cells (for example, human breast cancer cells (MCF7, MDA-MB-453), and the like), and the like.

"Fragmented extracellular matrix components" can be obtained by fragmenting extracellular matrix components. Extracellular matrix components are extracellular matrix molecule aggregates formed by a plurality of extracellular matrix molecules. The extracellular matrix molecule may be a substance that is present outside the cells in a multicellular organism. Arbitrary substances can be used as the extracellular matrix molecules as long as they do not adversely affect growth of cells and formation of cell aggregates. Examples of the extracellular matrix molecules include collagen, laminin, fibronectin, vitronectin, elastin, tenascin, entactin, fibrillin, proteoglycan, and the like, but are not limited thereto. As extracellular matrix components, these extracellular matrix molecules may be used alone or in combination of two or more thereof.

The extracellular matrix molecules may be modified extracellular matrix molecules or variants of extracellular matrix molecules or may be polypeptides such as chemically synthesized peptides. The extracellular matrix molecule may have a repetition of a sequence represented by Gly-X-Y, which is a characteristic of collagen. Here, Gly represents a glycine residue, and X and Y each independently represent any amino acid residue. A plurality of Gly-X-Y's may be the same as or different from each other. By having the repetition of the sequence represented by Gly-X-Y, there are fewer constraints on the arrangement of molecular chains. In the extracellular matrix molecule having the repetition of the sequence represented by Gly-X-Y, the proportion of the sequence represented by Gly-X-Y may be 80% or more and preferably 95% or more of the complete amino acid sequence. In addition, the extracellular matrix molecule may be a polypeptide having an RGD sequence. The RGD sequence refers to a sequence represented by Arg-Gly-Asp (arginine residue-glycine residue-aspartic acid residue). Examples of the extracellular matrix molecule containing a sequence represented by Gly-X-Y and an RGD sequence include collagen, fibronectin, vitronectin, laminin, and cadherin.

Examples of the collagen include fibrous collagen and non-fibrous collagen. Fibrous collagen refers to collagen containing collagen fibers as a main component, and specific examples thereof include type I collagen, type II collagen, type III collagen, and the like. Examples of the non-fibrous collagen include type IV collagen, and the like. The collagen is preferably fibrillar collagen.

Examples of the proteoglycans include chondroitin sulfate proteoglycan, heparan sulfate proteoglycan, keratan sulfate proteoglycan, and dermatan sulfate proteoglycan, but the present invention is not limited thereto.

The extracellular matrix component may contain at least one selected from the group consisting of collagen, laminin and fibronectin, and preferably contains collagen from the viewpoint of excellent cell adhesiveness. The collagen is preferably fibrous collagen, and more preferably type I collagen. Regarding the fibrous collagen, commercially available collagen may be used, and specific examples thereof include porcine skin-derived type I collagen (manufactured by NH Foods Ltd.).

The extracellular matrix component may be derived from animals. Examples of animal species from which an extracellular matrix component is derived include humans, pigs, and cows, but the present invention is not limited thereto. Regarding the extracellular matrix component, a component derived from one kind of animal may be used, or a component derived from a plurality of kinds of animals may be used in combination.

In this specification, "fragmentation" refers to making the aggregate of extracellular matrix molecules smaller in size. Fragmentation may be performed under conditions in which the bond within the extracellular matrix molecule breaks or may be performed under conditions in which the bond within the extracellular matrix molecule does not break. The fragmented extracellular matrix components may include defibrated extracellular matrix components in which the above-described extracellular matrix components are defibrated through application of physical force. Defibrillation is an aspect of fragmentation, and may be performed, for example, under conditions in which the bond within the extracellular matrix molecule does not break.

A method for fragmenting an extracellular matrix component is not particularly limited. Regarding a method for defibrating an extracellular matrix component, for example, an extracellular matrix component may be defibrated by applying a physical force with an ultrasonic homogenizer, a stirring homogenizer, a high pressure homogenizer or the like. When the stirring homogenizer is used, the extracellular matrix component may be homogenized directly or may be homogenized in an aqueous medium such as saline. In addition, a defibrated extracellular matrix component with a millimeter size or nanometer size can also be obtained by adjusting a homogenizing time, the number of times, and the like. The defibrated extracellular matrix component can also be obtained according to defibrating by repeating freezing and melting.

At least some of the fragmented extracellular matrix components may contain the defibrated extracellular matrix components. In addition, the fragmented extracellular matrix components may consist of only defibrated extracellular matrix components. That is, the fragmented extracellular matrix component may be a defibrated extracellular matrix component. The defibrated extracellular matrix component preferably contains a defibrated collagen component. The defibrated collagen component preferably maintains a triple helix structure derived from collagen. The defibrated collagen component may be a component that completely or partially maintains a triple helix structure derived from collagen.

Examples of the shape of the fragmented extracellular matrix component include a fibrous shape. The fibrous shape is a shape configured with a filamentous fragmented extracellular matrix component or a shape configured with filamentous fragmented extracellular matrix components cross-linked between molecules. At least some of the fragmented extracellular matrix components may have a fibrous shape. Examples of the fibrous extracellular matrix components include fine filaments (fine fibers) formed by aggregating a plurality of filamentous extracellular matrix molecules, filaments formed by further aggregating fine fibers, and those obtained by defibrating these filaments. The fibrous extracellular matrix component can retain the RGD sequence without disruption.

An average length of the fragmented extracellular matrix components may be 100 nm or more and 400 µm or less or may be 100 nm or more and 200 µm or less. In one embodiment, an average length of the fragmented extracellular matrix components may be 5 µm or more and 400 µm or less, may be 10 µm or more and 400 µm or less, may be 22 µm or more and 400 µm or less, or may be 100 µm or more and 400 µm or less. In another embodiment, an average length of the fragmented extracellular matrix components may be 100 µm or less, may be 50 µm or less, may be 30 µm or less, may be 15 µm or less, may be 10 µm or less, may be 1 µm or less, or may be 100 nm or more. An average length of the majority of the fragmented extracellular matrix components among all the fragmented extracellular matrix components may be within the above numerical ranges. Specifically, an average length of 95% of all the fragmented extracellular matrix components may be within the above numerical ranges. The fragmented extracellular matrix components may be fragmented collagen components having an average length within the above ranges, or may be defibrated collagen components having an average length within the above ranges.

An average diameter of the fragmented extracellular matrix components may be 10 nm or more and 30 µm or less, may be 30 nm or more and 30 µm or less, may be 50 nm or more and 30 µm or less, may be 100 nm or more and 30 µm or less, may be 1 µm or more and 30 µm or less, may be 2 µm or more and 30 µm or less, may be 3 µm or more and 30 µm or less, may be 4 µm or more and 30 µm or less, or may be 5 µm or more and 30 µm or less. The fragmented extracellular matrix components are preferably fragmented collagen components having an average diameter within the above ranges, and more preferably defibrated collagen components having an average diameter within the above ranges.

The average length and average diameter of the fragmented extracellular matrix components can be obtained by measuring each fragmented extracellular matrix component using an optical microscope and performing image analysis. In this specification, the "average length" refers to an average value of the lengths of measured samples in the longitudinal direction, and the "average diameter" refers to an average value of the lengths of measured samples in a direction orthogonal to the longitudinal direction.

The fragmented extracellular matrix components may contain, for example, fragmented collagen components, or may be configured with fragmented collagen components. The "fragmented collagen components" are components which are obtained by fragmenting collagen components such as fibrous collagen components, and which maintain a triple helix structure. An average length of the fragmented collagen components is preferably 100 nm to 200 µm, more preferably 22 µm to 200 µm, and still more preferably 100 µm to 200 µm. An average diameter of the fragmented collagen components is preferably 50 nm to 30 µm, more preferably 4 µm to 30 µm, and still more preferably 20 µm to 30 µm.

At least some of the fragmented extracellular matrix components may be cross-linked between molecules and within molecules. The extracellular matrix components may be cross-linked within extracellular matrix molecules or between extracellular matrix molecules configuring the extracellular matrix components.

Examples of a cross-linking method include a physical cross-linking method by applying heat, ultraviolet rays, radiation, and the like, and a chemical cross-linking method using a cross-linking agent, an enzyme reaction and the like, and the method is not particularly limited. Physical cross-linking is preferable from the viewpoint of not interfering with cell growth. Cross-linking (physical cross-linking and chemical cross-linking) may be cross-linking via covalent bonds.

When the extracellular matrix components contain collagen components, cross-links may be formed between collagen molecules (triple helix structure), and may be formed between collagen fibrils formed by collagen molecules. Cross-linking may be cross-linking by heat (thermal cross-linking). Thermal cross-linking can be performed, for example, by performing a heat treatment under a reduced pressure using a vacuum pump. When the collagen components are thermally cross-linked, the extracellular matrix components may be cross-linked when amino groups of collagen molecules form peptide bonds (-NH-CO-) with carboxyl groups of the same or other collagen molecules.

The extracellular matrix components can be cross-linked using a cross-linking agent. The cross-linking agent can, for example, cross-link carboxyl groups and amino groups, or can cross-link amino groups with each other. As the cross-linking agent, for example, an aldehyde-based, carbodiimide-based, epoxide-based or imidazole-based cross-linking agent is preferable from the viewpoint of cost, safety and operability, and specific examples thereof include water-soluble carbodiimides such as glutaraldehyde, 1-ethyl-3-(3-dimethylaminopropyl)carbodiimide/hydrochloride, and 1-cyclohexyl-3-(2-morpholinyl-4-ethyl)carbodiimide/sulfonate.

Quantification of the degree of cross-linking can be appropriately selected in accordance with the type of the extracellular matrix components, a method for cross-linking, and the like. The degree of cross-linking may be 1% or more, 2% or more, 4% or more, 8% or more, or 12% or more, and may be 30% or less, 20% or less, or 15% or less. When the degree of cross-linking is within the above ranges, the extracellular matrix molecule can be appropriately dispersed, and the redispersibility after dry storage is favorable.

When amino groups in the extracellular matrix components are used for cross-linking, the degree of cross-linking can be quantified on the basis of a TNBS (trinitrobenzenesulfonic acid) method. The degree of cross-linking by the TNBS method may be within the above ranges. The degree of cross-linking by the TNBS method is a proportion of amino groups used for cross-linking among amino groups of the extracellular matrix. When the extracellular matrix components contain collagen components, the degree of cross-linking measured by the TNBS method is preferably within the above ranges.

The degree of cross-linking may be calculated by quantifying carboxyl groups. For example, in the case of extracellular matrix components that are insoluble in water, quantification may be performed by a TBO (toluidine blue O) method. The degree of cross-linking by the TBO method may be within the above ranges.

The content of the extracellular matrix components in the three-dimensional tissue may be 0.01 to 90% by mass, preferably 10 to 90% by mass, preferably 10 to 80% by mass, preferably 10 to 70% by mass, preferably 10 to 60% by mass, preferably 1 to 50% by mass, preferably 10 to 50% by mass, more preferably 10 to 30% by mass, and more preferably 20 to 30% by mass, on the basis of the three-dimensional tissue (dry weight).

Here, the "extracellular matrix components in the three-dimensional tissue" refers to extracellular matrix components that configure the three-dimensional tissue, and may be derived from endogenous extracellular matrix components or exogenous extracellular matrix components.

The "endogenous extracellular matrix components" are extracellular matrix components produced from extracellular matrix-producing cells. Examples of the extracellular matrix-producing cells include mesenchymal cells such as fibroblasts, chondrocytes, and adipocytes. The endogenous extracellular matrix components may be fibrous or non-fibrous.

The "exogenous extracellular matrix components" are extracellular matrix components supplied from the outside. The three-dimensional tissue according to this embodiment contains fragmented extracellular matrix components, which are exogenous extracellular matrix components. The exogenous extracellular matrix components may be the same as or different from endogenous extracellular matrix components of the animal species from which they are derived. Examples of the originating animal species include humans, pigs, and cows. In addition, the exogenous extracellular matrix components may be artificial extracellular matrix components.

In other words, when the three-dimensional tissue contains endogenous extracellular matrix components and fragmented extracellular matrix components, the content of the extracellular matrix components configuring the three-dimensional tissue refers to the total amount of endogenous extracellular matrix components and fragmented extracellular matrix components. The extracellular matrix content can be calculated from the volume of the obtained three-dimensional tissue and the mass of decellularized three-dimensional tissue.

When the extracellular matrix components are collagen components, the exogenous extracellular matrix components are also referred to as "exogenous collagen components", and the "exogenous collagen components", which are collagen components supplied from the outside, are aggregates of collagen molecules formed by a plurality of collagen molecules, and specific examples thereof include fibrous collagen, non-fibrous collagen, and the like. The exogenous collagen component is preferably fibrous collagen. The fibrous collagen refers to a collagen component which is a main component of collagen fibers, and examples thereof include type I collagen, type II collagen, and type III collagen. As the fibrous collagen, commercially available collagen may be used, and specific examples thereof include porcine skin-derived type I collagen (manufactured by NH Foods Ltd.). Examples of the exogenous non-fibrous collagen include type IV collagen.

In the exogenous extracellular matrix components, the originating animal species may be different from that of the cells. In addition, when the cells include extracellular matrix-producing cells, in the exogenous extracellular matrix components, the originating animal species may be different from that of the extracellular matrix-producing cells. That is, the exogenous extracellular matrix components may be heterologous extracellular matrix components.

For example, when the extracellular matrix components contained in the three-dimensional tissue are collagen components, a method for quantifying the amount of collagen components in the three-dimensional tissue can be, for example, a method for quantifying hydroxyproline as follows. A sample is prepared by mixing hydrochloric acid (HCl) with a solution dissolved in the three-dimensional tissue, incubating at a high temperature for a predetermined period of time, and then returning it to room temperature, and diluting the centrifuged supernatant to a predetermined concentration. A hydroxyproline standard solution is treated in the same manner as the sample, and then gradually diluted to prepare a standard. Each of the sample and the standard is subjected to a predetermined treatment with a hydroxyproline assay buffer and a detection reagent, and the absorbance at 570 nm is measured. The amount of the collagen components is calculated by comparing the absorbance of the sample with that of the standard. Note that the three-dimensional tissue may be directly suspended in high-concentration hydrochloric acid, the dissolved solution may be centrifuged, and the supernatant may be recovered and used for quantifying the collagen components. Furthermore, the three-dimensional tissue to be dissolved may be used in a state of being recovered from a culture medium, or may be dissolved by performing a drying treatment after recovery to remove the liquid components. However, when the three-dimensional tissue in a state of being recovered from the culture medium is dissolved to quantify the collagen components, it is expected that a measured value of the weight of the three-dimensional tissue will vary due to the influence of medium components absorbed by the three-dimensional tissue and the remaining medium due to problems with the experimental technique, and thus it is preferable to use the weight after drying as a standard from the viewpoint of stably measuring the weight of the structure and the amount of the collagen components per unit weight.

A more specific example of a method for quantifying the amount of the collagen components is, for example, the following method.

### (Preparation of sample)

A total amount of the three-dimensional tissue having been subjected to a freeze-drying treatment is mixed with 6 mol/L HCl and incubated in a heat block at 95°C for 20 hours or more, and then returned to room temperature. After centrifugation is performed at 13,000 g for 10 minutes, the supernatant of the sample solution is recovered. A sample is prepared by appropriately diluting the supernatant with 6 mol/L HCl so that the result falls within a range of a calibration curve in the measurement to be described below, and then performing dilution of 200 µL with 100 µL of ultrapure water. 35 µL of the sample is used.

### (Preparation of standard)

125 µL of a standard solution (1,200 µg/mL in acetic acid) and 125 µL of 12 mol/l HCl are put into a screw cap tube and mixed, the mixture is incubated at 95°C for 20 hours in a heat block, and the temperature is then returned to room temperature. After centrifugation is performed at 13,000g for 10 minutes, the supernatant is diluted with ultrapure water to prepare S1 of 300 µg/mL, and S1 is diluted in a stepwise manner to prepare S2 of (200 µg/mL), S3 (100 µg/mL), S4 (50 µg/mL), S5 (25 µg/mL), S6 (12.5 µg/mL), and S7 (6.25 µg/mL). S8 (0 µg/mL) containing only 90 µL of 4 mol/L HCl is also prepared.

### (Assay)

35 µL of the standard and the sample are put into a plate (included in QuickZyme Total Collagen Assay kit, made by QuickZyme Biosciences). 75 µL of an assay buffer (included in the above-mentioned kit) is added to each well. The plate is closed with a seal, and incubation is performed at room temperature while shaking it for 20 minutes. The seal is removed, and 75 µL of a detection reagent (reagent A:B = 30 µL:45 µL, included in the above-mentioned kit) is added to each well. The plate is closed with a seal, the solution is mixed by shaking, and incubation is performed at 60°C for 60 minutes. Cooling is sufficiently performed on ice, the seal is removed, and the absorbance at 570 nm is measured. The amount of the collagen components is calculated by comparing the absorbance of the sample with that of the standard.

The collagen components occupying the three-dimensional tissue may be defined by the area ratio or volume ratio thereof. "Defining by the area ratio or the volume ratio" means that, for example, the collagen components in the three-dimensional tissue are made distinguishable from other tissue structures by a known staining method (for example, immunostaining using anti-collagen antibodies or Masson trichrome staining), and a ratio of a region where the collagen components are present relative to the entire three-dimensional tissue is then calculated using observation with the naked eye, various microscopes, image analysis software and the like. When defining the collagen components by the area ratio, there is no limitation on which cross section or surface in the three-dimensional tissue is used to define the area ratio. However, for example, when the three-dimensional tissue is a sphere or the like, the collagen components may be defined by a cross section passing through substantially the center thereof.

For example, when the collagen components in the three-dimensional tissue are defined by the area ratio, the proportion of the area is 0.01 to 99% on the basis of the total area of the three-dimensional tissue, preferably 1 to 99%, more preferably 5 to 90%, more preferably 7 to 90%, more preferably 20 to 90%, and more preferably 50 to 90%. The "collagen components in the three-dimensional tissue" is as described above. The proportion of the area of the collagen components configuring the three-dimensional tissue refers to the proportion of the combined area of the endogenous collagen components and the exogenous collagen components. The proportion of the area of the collagen components can be calculated, for example, by staining the obtained three-dimensional tissue with Masson trichrome and calculating the proportion of the area of the collagen components stained blue to the total area of the cross section passing through substantially the center of the three-dimensional tissue.

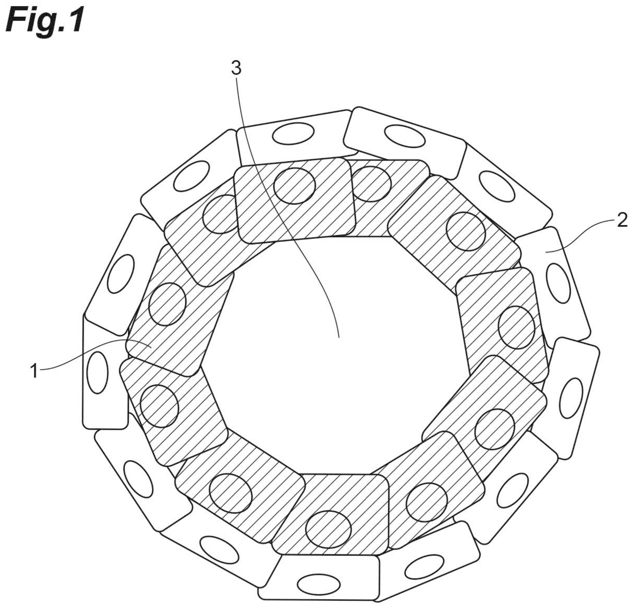

The three-dimensional tissue according to this embodiment has a void surrounded by the two layers of mammary epithelial cells. The voids surrounded by the two layers of mammary epithelial cells according to one embodiment are shown in FIG. 1. As differentiation progresses, the mammary epithelial cells in the three-dimensional tissue according to one embodiment form a ring-shaped layer consisting of mammary epithelial cells 1 on a luminal side (inside) and a ring-shaped layer consisting of mammary epithelial cells 2 on a basement membrane side (outside). Thereby, a void 3 surrounded by the two layers of mammary epithelial cells is formed.

It is preferable that the mammary epithelial cells 1 and 2 in the void 3 surrounded by the two layers of mammary epithelial cells are, for example, CK8/18 positive cells in the mammary epithelial cells 1 on the luminal side and CK14 positive cells in the mammary epithelial cells 2 on the basement membrane side. In this specification, "positive cells" refer to cells that express a specific marker protein on the cell surface or intracellularly. For example, "CK8/18 positive cells" refer to cells that express CK8 protein or CK18 protein. CK8/18 and CK14 are differentiation markers identified in living mammary gland tissue, and such a two-layer structure is also found in living mammary glands, making this more suitable for a mammary gland model than in this case.

The three-dimensional tissue according to this embodiment may further contain a hydrogel. In this specification, "hydrogel" refers to a three-dimensional mesh structure formed by cross-linking polymers through hydrogen bonds, ionic bonds, coordinate bonds, covalent bonds, and the like, and containing a liquid such as water inside the three-dimensional mesh structure. When the three-dimensional tissue according to this embodiment contains a hydrogel, shrinkage of the three-dimensional tissue is promoted, and the shape of the three-dimensional tissue becomes spherical, similar to the shape of mammary tissue in a living body.

Examples of the hydrogel include fibrin gel, collagen gel, gelatin gel, hyaluronic acid gel, alginate gel, pectin gel, and the like, but are not limited thereto. The hydrogel is preferably a fibrin gel. Fibrin is a component produced by the action of thrombin on fibrinogen to release Aα chains, A chains from the N terminal of Bβ chains, and B chains. Fibrin is formed by bringing fibrinogen into contact with thrombin. The hydrogel may consist of one of the above-mentioned substances, or may contain two or more of them in combination.

When the three-dimensional tissue according to this embodiment contains a hydrogel, the three-dimensional tissue may or may not be embedded in the hydrogel, but from the viewpoint of promoting shrinkage more, it is preferable that the three-dimensional tissue is embedded. "The three-dimensional tissue is embedded in the hydrogel" means that the hydrogel is present in at least some or all of the intercellular spaces outside or both outside and inside the three-dimensional tissue.

The three-dimensional tissue according to this embodiment is obtained by co-culturing mature adipocytes and mammary epithelial cells, and since differentiation of the mammary epithelial cells is progressing, a large amount of lipid droplets can be accumulated in the three-dimensional tissue. In this specification, "a large amount of lipid droplets can be accumulated" means that the area of a region where lipid droplets are present in an observation image acquired when the lipid droplets in the three-dimensional tissue are stained with a fluorescent substance capable of detecting lipid droplets, such as Nile red, and the three-dimensional tissue is observed using a microscope may be 30% or more, 40% or more, 50% or more, 60% or more, 70% or more, or 80% or more of the area of the three-dimensional tissue, or may be 95% or less, 90% or less, 85% or less, 80% or less, or 75% or less. The area of the region where lipid droplets are present is not limited by the cross section or surface in the three-dimensional tissue that defines the area, but for example, when the three-dimensional tissue is a sphere or the like, it may be defined by a cross section passing through substantially the center of the three-dimensional tissue.

In the three-dimensional tissue according to this embodiment, differentiation of mammary epithelial cells is progressing, and thus differentiation markers such as CK8/18 and CK14 that are identified in living mammary tissue are expressed in the three-dimensional tissue. The three-dimensional tissue according to this embodiment is immunostained for CK8/18 or CK14, and in the observation image obtained when the three-dimensional tissue is observed using a microscope, the area of the region in which CK8/18 or CK14 is identified may be 20% or more, 30% or more, 40% or more, 50% or more, 60% or more, 70% or more, or 80% or more of the area of the three-dimensional tissue, or may be 95% or less, 90% or less, 85% or less, 80% or less, or 75% or less.

As described above, the three-dimensional mammary gland model according to this embodiment is capable of obtaining milk. "Capable of obtaining milk" may mean that milk proteins contained in milk are detected in the three-dimensional tissue configuring the three-dimensional mammary gland model. "Milk proteins are detected" may mean, for example, that the amount of milk proteins is 1.5 times or more, 3 times or more, 4 times or more, 5 times or more, 6 times or more, 7 times or more, 8 times or more, 9 times or more, or 10 times or more compared to the amount of milk proteins in two-dimensionally cultured mammary epithelial cells.

The amount of milk proteins in the three-dimensional tissue is not particularly limited as long as it can be measured, but can be measured, for example, by a method to be described in examples below, and more specifically, by Western blotting or the like.

Examples of the milk proteins include casein, lactoferrin, lactalbumin, lactoglobulin, and the like. The milk proteins detected in the three-dimensional tissue according to this embodiment may be one of the above-mentioned types, or two or more types.

The three-dimensional tissue according to this embodiment may exhibit responsiveness to a substance that stimulates milk secretion. "Exhibiting responsiveness to a substance that stimulates milk secretion" may mean that the amount of milk proteins is increased compared to when milk secretion is not stimulated, and may mean that the amount of milk proteins is increased, for example, by 1.1 times or more, 1.2 times or more, 1.3 times or more, 1.4 times or more, or 1.5 times or more.

Examples of the substance that stimulates milk secretion include prolactin, oxytocin, and the like.

The three-dimensional tissue according to this embodiment may be one in which, for example, after being cultured for 7 days in a medium containing 1 µg/mL of prolactin, when casein protein in the three-dimensional tissue is detected by Western blotting, the band brightness of the casein protein relative to α-tubulin is detected as 1% or more, 5% or more, 10% or more, 20% or more, 30% or more, or 40% or more, or is detected as 300% or less, 200% or less, or 100% or less.

The three-dimensional tissue according to the present embodiment may exhibit an increased expression level of claudin 3 (CLDN3) when analyzed by quantitative PCR after being cultured for 7 days in a medium containing, for example, 1 µg/mL of prolactin, as compared to the expression level of CLDN3 in the three-dimensional tissue cultured for 7 days in a medium not containing prolactin. The increase may be, for example, 1.05 times or more, 1.1 times or more, 1.15 times or more, 1.2 times or more, 1.25 times or more, or 1.3 times or more. Here, CLDN3 is one of the claudins, which are four-pass transmembrane proteins that configure a tight junction, which is a type of intercellular junction.

The thickness of the three-dimensional tissue may be 10 µm or more, 30 µm or more, 50 µm or more, 100 µm or more, 300 µm or more, or 1000 µm or more. Such a three-dimensional tissue has a structure closer to that of a biological tissue, and is suitable as a substitute for experimental animals and a grafting material. The upper limit of the thickness of the three-dimensional tissue is not particularly limited, but may be, for example, 10 mm or less, 3 mm or less, 2 mm or less, 1.5 mm or less, 1 mm or less, 300 µm or less, 200 µm or less, 150 µm or less, or 100 µm or less.

Here, "the thickness of the three-dimensional tissue" refers to a distance between both ends in a direction perpendicular to the main surface when the three-dimensional tissue is a rectangular parallelepiped. When the main surface is uneven, the thickness refers to a distance at the thinnest part of the main surface.

When the three-dimensional tissue is spherical or substantially spherical, the thickness of the three-dimensional tissue refers to the diameter of the three-dimensional tissue. When the three-dimensional tissue is ellipsoidal or substantially ellipsoidal, the thickness of the three-dimensional tissue refers to the minor axis of the three-dimensional tissue. When the three-dimensional tissue is substantially spherical or substantially ellipsoidal and has an uneven surface, the thickness of the three-dimensional tissue refers to the shortest distance between two points where a straight line passing through the center of gravity of the three-dimensional tissue intersects with the above-mentioned surface.

The three-dimensional tissue according to this embodiment is constructed in a cell culture vessel. There are no particular limitations on the cell culture vessel, as long as it is a vessel in which a three-dimensional tissue can be constructed and the constructed three-dimensional tissue can be cultured. Specific examples of the cell culture vessel include dishes, cell culture inserts (for example, Transwell (registered trademark) inserts, Netwell (registered trademark) inserts, Falcon (registered trademark) cell culture inserts, Millicell (registered trademark) cell culture inserts, and the like), tubes, flasks, bottles, plates, and the like. When constructing the three-dimensional tissue, a dish or various cell culture inserts are preferred from the viewpoint of enabling evaluation using the three-dimensional tissue to be performed more appropriately.

[Method for producing three-dimensional mammary gland model] A method for producing a three-dimensional mammary gland model according to this embodiment includes a step of bringing cells into contact with fragmented extracellular matrix components in an aqueous medium (contact step), and a step of culturing the cells contacting the fragmented extracellular matrix components (culture step). The cells contain mature adipocytes, adipose-derived stem cells, and mammary epithelial cells, as described above.

The method for producing the three-dimensional mammary gland model according to this embodiment has the above-mentioned configuration, which makes it possible to co-culture mammary epithelial cells and mature adipocytes and promotes differentiation of mammary epithelial cells, thereby making it possible to easily produce a mammary gland tissue model from which milk can be obtained.

In the contact step, mature adipocytes, adipose-derived stem cells, mammary epithelial cells, and fragmented extracellular matrix components are contacted in an aqueous medium.

A ratio (number of cells) of mature adipocytes: mammary epithelial cells in the contact step is as described above. In addition, the ratio (number of cells) of mature adipocytes: adipose-derived stem cells in the contact step may be 1:1 to 2.5:1, or may be 1.2:1 to 2:1. In addition, the ratio (number of cells) of adipose-derived stem cells: mammary epithelial cells in the contact step may be 1:0.5 to 1:4, or may be 1:0.5 to 1:3, or may be 1:0.5 to 1:2, or may be 1:0.5 to 1:1, or may be 1:1 to 1:2.

The "aqueous medium" refers to a liquid containing water as an essential component. There are no particular limitations on the aqueous medium, as long as the fragmented extracellular matrix components can be present stably in the medium. Specific examples of the aqueous medium include physiological saline such as phosphate-buffered saline (PBS), and liquid media such as a Dulbecco's Modified Eagle medium (DMEM) and a mammary epithelial cell medium (MEGM), but are not limited thereto. The liquid medium may be a mixed medium in which two types of media have been mixed.

Examples of a method for bringing cells into contact with fragmented extracellular matrix components include a method for mixing an aqueous medium containing the fragmented extracellular matrix components with a culture medium containing cells, a method for adding an aqueous medium containing the fragmented extracellular matrix components to a culture medium containing cells, a method for adding cells to an aqueous medium containing the fragmented extracellular matrix components, and a method for adding the fragmented extracellular matrix components and the cells to a previously prepared aqueous medium.

The order in which the cells are brought into contact with the fragmented extracellular matrix components is not particularly limited, and for example, some cells may be brought into contact with the fragmented extracellular matrix components, and the remaining cells may be then brought into contact with the fragmented extracellular matrix components, or all cells may be brought into contact with the fragmented extracellular matrix components simultaneously or substantially simultaneously. From the viewpoint of facilitating the production of the three-dimensional tissue, it is preferable to bring the adipose-derived stem cells, the mammary epithelial cells, and the fragmented extracellular matrix components into contact with each other, and then to bring the mature adipocytes into contact with the fragmented extracellular matrix components.

An aqueous medium containing cells and fragmented extracellular matrix components may or may not be mixed by, for example, stirring after each substance is added. The contact step may include bringing the cells and the fragmented extracellular matrix components into contact with each other and then incubating them for a certain period of time.

The concentration of the fragmented extracellular matrix components in the contact step can be appropriately determined in accordance with the shape and thickness of the target three-dimensional tissue, the size of an incubator, and the like. For example, the concentration of the fragmented extracellular matrix components in the aqueous medium in the contact step may be 0.1 to 90% by mass, or may be 1 to 30% by mass.

The amount of the fragmented extracellular matrix components in the contact step may be, for example, 0.1 to 100 mg, 0.5 to 50 mg, 0.8 to 25 mg, 1.0 to 10 mg, 1.0 to 5.0 mg, 1.0 to 2.0 mg, or 1.0 to 1.8 mg relative to 1.0 x 10⁶ cells, may be 0.7 mg or more, 1.1 mg or more, 1.2 mg or more, 1.3 mg or more, or 1.4 mg or more, or may be 7.0 mg or less, 3.0 mg or less, 2.3 mg or less, 1.8 mg or less, 1.7 mg or less, 1.6 mg or less, or 1.5 mg or less.

In the contact step, the mass ratio of the fragmented extracellular matrix components to the cells (fragmented extracellular matrix components/cells) is preferably 1/1 to 1,000/1, more preferably 9/1 to 900/1, and still more preferably 10/1 to 500/1.

In the culture step, the cells brought into contact with the fragmented extracellular matrix components are cultured to form a three-dimensional tissue.

Examples of the medium used in the culture step include a mammary epithelial cell medium (for example, a MEGM medium (manufactured by Lonza)), a KBM medium, and the like. The medium may be a medium to which serum is added, or may be a serum-free medium. The medium may be a medium supplemented with growth factors, hormones such as insulin, and the like. The medium may be a mixed medium in which two types of mediums are mixed.

The culture temperature in the culture step may be, for example, 20°C to 40°C, or may be 30°C to 37°C. The pH of the medium may be 6 to 8, or may be 7.2 to 7.4. The culture time may be 1 day to 2 weeks, or may be 1 week to 2 weeks.

The incubator (support) is not particularly limited, and may be, for example, a dish, a well insert, a low adhesive plate, or a plate having a bottom shape such as a U shape, and a V shape. The cells may be cultured while it is adhered to the support, or the cells may be cultured without being adhered to the support or may be separated from the support during culture and cultured. When the cells are cultured without being adhered to the support or are separated from the support during culture and cultured, it is preferable to use a plate having a bottom shape such as a U shape and a V shape that inhibits adhesion of the cells to the support or a low adsorption plate.

The cell density in the medium in the culture step can be appropriately determined depending on the shape and thickness of the target three-dimensional tissue, the size of the incubator, and the like. For example, the cell density in the medium in the culture step may be 1 to 10⁸ cells/mL or may be 10³ to 10⁷ cells/mL. In addition, the cell density in the medium in the culture step may be the same as the cell density in the aqueous medium in the contact step.

The producing method according to this embodiment may further include a step of bringing a hydrogel precursor into contact in the contact step, and gelling the hydrogel precursor after the contact step and before the culture step (gelling step).

A hydrogel precursor is a substance that forms a hydrogel in response to some external stimulus, such as a chemical or physical stimulus, and is a substance that has not yet formed a hydrogel. Hydrogels include those mentioned above, and hydrogel precursors include, for example, fibrinogen, collagen, gelatin, hyaluronic acid, alginic acid, and pectin. The hydrogel precursor preferably contains fibrinogen. Fibrinogen forms a fibrin gel by reacting with thrombin. Thus, it is sufficient that fibrinogen and thrombin come into contact with each other in the aqueous medium in the contact step or gelling step. For example, when the aqueous medium contains serum, thrombin is contained in the serum, and thus a fibrin gel can be formed without adding thrombin.

The concentration of the hydrogel precursor in the aqueous medium in the contact step may be 0.1 mg/mL or more, 0.2 mg/mL or more, 0.3 mg/mL or more, 0.4 mg/mL or more, 0.5 mg/mL or more, 0.6 mg/mL or more, 0.7 mg/mL or more, 0.8 mg/mL or more, 0.9 mg/mL or more, 1.0 mg/mL or more, or 2.0 mg/mL or more, on the basis of the total amount of the aqueous medium. The concentration of the hydrogel precursor in the aqueous medium may be 10.0 mg/mL or less, 8.0 mg/mL or less, 7.0 mg/mL or less, 6.0 mg/mL or less, 5.0 mg/mL or less, 3.0 mg/mL or less, 1.0 mg/mL or less, 0.8 mg/mL or less, or 0.6 mg/mL or less, on the basis of the total amount of the aqueous medium.

For example, when the hydrogel precursor contains two or more substances, such as a combination of fibrinogen and thrombin, the contact step may be obtained by mixing, for example, a first solution containing cells, fragmented extracellular matrix components, an aqueous medium, and a first hydrogel precursor (for example, fibrinogen) with a second solution containing an aqueous medium and a second hydrogel precursor (for example, thrombin). Note that the second solution may contain cells and/or fragmented extracellular matrix components, or both the first solution and the second solution may contain cells and/or fragmented extracellular matrix components.

When a hydrogel precursor is further brought into contact in the contact step, the order in which the cells, the fragmented extracellular matrix components, and the hydrogel precursor are brought into contact is not particularly limited. From the viewpoint of facilitating the production of the three-dimensional tissue, the contact step is preferably, for example, such that the adipose-derived stem cells, the mammary epithelial cells, and the fragmented extracellular matrix components are brought into contact, and then the mature adipocytes and the hydrogel precursor are brought into contact with the fragmented extracellular matrix components. When the hydrogel precursor contains two or more types of substances, it is preferable to bring the adipose-derived stem cells, the mammary epithelial cells, the fragmented extracellular matrix components, the mature adipocytes, and the first hydrogel precursor into contact in the same order, and then bring the first hydrogel precursor into contact with the second hydrogel precursor.

The gelling step is a step of forming a hydrogel containing cells and fragmented extracellular matrix components. The gelling step may include performing incubation for a certain period of time. The gelling step may be a step of embedding the cells and the fragmented extracellular matrix components in a hydrogel.

### [Example]

Hereinafter, the present invention will be described in more detail with reference to examples. However, the present invention is not limited to the following examples.

The cells and reagents used in preparing the three-dimensional mammary gland model are listed in Tables 1 and 2 below. A method for preparing CMF and a method for collecting adipose-derived human stem cells and mature adipocytes are also described below.

**[Table 1]**

| Cells | |
|---|---|
| Product Name | Model Number |
| Mammary epithelial cell (HMEC) | CC-2551 |
| Adipose-derived human stem cell (hADSC) | Isolated from human adipose tissue extracted by surgery or the like using method described below. |

**[Table 2]**

| Medium, reagent, various experimental equipment | | | |
|---|---|---|---|
| Abbreviation | Product Name | Manufacturer | Model Number |
| DMEM | DMEM (high glucose) (glutamine, pyruvate-containing X liquid) for cell culture Capacity: 10 x 500 ML | Nacalai Tesque | 0845816 |
| PBS | Dulbecco's Phosphate Buffered Saline (not containing Ca, Mg) (liquid) Capacity: 10 x 500M | Nacalai Tesque | 1424924 |
| KBM | KBEM-VEC1 | Kohiin Bio | 16030100 |
| MEGM | Bullet Kit MEGM Growth medium for mammary epithelial cells | Lonza | cc-3150 |
| Antibiotic | Antibiotic-antifungal mixture (100x concentrated) for cell culture Capacity: 100ML | Nacalai Tesque | 0289254 |
| Trypsin | Trypsin (Made from pork innards) Capacity: 10G | Nacalai Tesque | 3554764 |
| Collagenase | Collagenase from Clostridium | Sigma-Aldrich | C0130 |
| | histolyticum | | |
| Fibrinogen | Thrombin from bovine plasma lyophilized powder | Sigma-Aldrich | T4648 |
| Insulin | Insulin from bovine pancreas | Sigma-Aldrich | C0130 |
| BSA | Bovine serum albumin (derived from bovine serum) | Sigma-Aldrich | A3294 |
| Collagen | Collagen Type I, Bovine Skin, Acid Soluble (3mg/ml) | NIP | 892171 |
| 96-well round-bottom plate | EZ-BindShut (registered trademark) SP 96 well (U bottom) plate | IWAKI | 4870-800SP |
| 24-well plate | Tissue culture microplate, 24 wells, flat bottom Capacity: 50 plates | IWAKI | 3820-024 |
| Schale | Tissue culture dish (for adhesive cells, surface treatment agent) 100mm Capacity: 300 pieces | IWAKI | 3020-100 |
| Anti-CK8/18 antibody | Monoclonal Rabbit Anti-Human Cytokeratin 8/18, Clone EP17/EP30 | DAKO | M365201-2 |
| Anti-CK14 antibody | Cytokeratin 14 Monoclonal Antibody (LL002) | Thermo Fisher | MA511599 |
| Anti-casein antibody | Anti-Casein Polyclonal Antibody | Bioss Antibodies | bs-0813R |
| Anti-α-tubulin antibody | Alpha Tubulin Monoclonal Antibody (yOL1/34) | Thermo Fisher | MA1-80189 |
| Alexa Fluor (trademark) | Goat anti-Mouse IgG (H+L) Cross-Adsorbed | Thermo Fisher | A21235 |
| 647-conjugated goat anti-mouse IgG secondary antibody | Secondary Antibody, Alexa Fluor 647 | | |
| Alexa Fluor (trademark) 546-conjugated goat anti-rabbit IgG secondary antibody | Goat anti-Rabbit IgG (H+L) Cross-Adsorbed Secondary Antibody, Alexa Fluor (trademark) 546 | Thermo Fisher | A11010 |
| Alexa Fluor (trademark) 488-conjugated goat anti-rat IgG secondary antibody | Goat anti-Rat IgG (H+L) Cross-Adsorbed Secondary Antibody, Alexa Fluor (trademark) 488 | Thermo Fisher | A11006 |
| Goat anti-rabbit IgG StarBright (trademark) Blue 700 | Goat anti-rabbit IgG StarBright (trademark) Blue 700, 400 µl | BIO RAD | 12004161 |
| Hoechst | Hoechst 33342, Trihydrochloride, Trihydrate - 10 mg/mL Solution in Water | Thermo Fisher | H3570 |
| Nile Red | Nile Red | Tokyo Chemical Industry Co., Ltd. | N0659 |
| NucleroSpin | NucleoSpin (trademark) RNA/Protein | Takara Bio Inc. | 740933.5 |
| Blocking buffer | EveyBlot Blocking Buffer | BIO RAD | 12010020 |
| 10 X TBS-T | Tris-buffered saline (containing 0.05% surfactant) (10x concentrated) (pH 7.4) | Nacalai Tesque | 12749-21 |
| SDS buffer | 10x Tris/Glycine/SDS | BIO RAD | 1610732 |
| Transfer buffer | Trans-Blot Turbo 5 X Transfer Buffer | BIO RAD | 10026938 |

### <Preparation of CMF>

A neutralization buffer containing 2.125 mL of 0.05N NaOH and 2.125 mL of 10x PBS was added to 17 mL of 3 mg/mL collagen solution, and the mixture was heated at 37°C for 30 minutes to cause gelling. The resulting gel was freeze-dried for 72 hours to obtain freeze-dried collagen after gelling. 15 mL of 85 v/v% ethanol was added to the freeze-dried collagen after gelling, and the mixture was defibrated for 6 minutes using a homogenizer (probe: S10N-10G-ST shaft generator). Samples obtained after defibration were centrifuged at 10,000 rpm, the supernatant was discarded, and 15 mL of 70 v/v % ethanol was added to each of the samples to perform pipetting using a homogenizer (S1 0N-8G shaft generator). The sample obtained after pipetting was centrifuged at 10,000 rpm, the supernatant was discarded, and 15 mL of ultrapure water was added and pipetted. Further, the sample was ultrasonicated 10 times for 20 seconds using an ultrasonic homogenizer. The sample containing defibrated collagen (CMF) obtained by the above procedure was freeze-dried while the CMF remained dispersed in water.

### <Collection of mature adipocytes and ADSC>

A human adipose tissue section was washed with PBS containing 5% antibiotics. 4 to 6 g of the tissue was split into 6 wells of a 6-well plate. In 2 mL of a 2 mg/mL collagenase solution, the sample was finely chopped into a size of approximately 1 to 3 mm using scissors and tweezers. After performing incubation at 37°C and 230 rpm for 30 minutes, the sample was pipetted using a 10 mL pipette. After further performing incubation for 30 minutes, 2 ml of a DMEM was added per well to stop the collagenase reaction. The dissolved material was filtered through a 500 µm mesh filter and centrifuged at 80 g for 3 minutes at room temperature (15 to 25°C). Mature adipocytes were included in an upper yellow oily layer, while adipose-derived stem cells and blood cells are contained in a pellet. A medium between upper and lower layers was aspirated and discarded using a 10 mL pipette, and the mature adipocytes contained in the upper layer and the adipose-derived stem cells and blood cells contained in the lower layer were washed twice with 25 mL of PBS (5% BSA, 1% antibiotics). During the washing, centrifuging was performed in the same manner as described above, and thus separation was performed into three layers: the upper layer, the lower layer, and the medium between the upper layer and the lower layer, and the medium between the upper layer and the lower layer was aspirated and discarded. After performing washing twice, washing with 25 mL of a DMEM was performed.

Only the upper layer including mature adipocytes was collected and dispensed into Eppendorf tubes. Nuclei were stained with Hoechst staining (Hoechst diluted 1,000-fold, staining for 15 minutes), and the number of cells on a Turker Burk hemacytometer was counted using a fluorescence microscope.

A pellet containing ADSC was suspended in 10 mL of a DMEM, seeded in a 10 cm dish, and sub-cultured. ADSCs were separated from the dish using trypsin/EDTA and suspended in 1 mL of a DMEM, and the number of cells was counted.

### [Test Example 1: Morphological confirmation by immunostaining in three-dimensional mammary gland model]

### <Production of three-dimensional mammary gland model>

hADSCs and HMECs that had been cultured in advance were collected, and the number of cells was counted and the number of cells required was calculated. A required amount of CMF was measured into an Eppendorf tube, 1 ml of Milli-Q was added, and the CMF was dispersed by pipetting. Thereafter, the mixture was centrifuged, and the supernatant was removed. The hADSC and HMEC cell suspension, whose the number of cells has been calculated, was added to the Eppendorf tube containing the CMF, the tube was centrifuged at room temperature for 1 minute without pipetting, and the supernatant was removed. Next, the required amount of fibrinogen was added to the Eppendorf tube and mixed without creating bubbles. The required amount of mature adipocytes was then separated and added to the Eppendorf tube, and gently mixed to prepare a cell suspension with the concentrations of each cell, CMF, and fibrinogen shown in Table 3. The prepared cell suspension was dispensed into 96-well round-bottom plates by 5 µL each (an amount for one tissue shown in Table 3). Thereafter, the mixture was incubated at 37°C and 5% CO₂ for 15 minutes to form a fibrin gel. 100 µL of a DMEM was added to each well, allowing the gel containing various cells and CMF to float. The floating gel was scooped up with a spoon and transferred to a 24-well plate containing 500 µL of a MEGM medium. The medium was changed every 2 to 3 days, and culture was performed for 7 days to produce a three-dimensional mammary gland model consisting of the three-dimensional tissue in Example 1.

**[Table 3]**

| | /1ml (blood clots) | /5µL (one tissue) |
|---|---|---|
| Mature adipocyte (mAD) | 3.5 × 10⁶ cells | 1.75 × 10⁴ cells |
| Adipose-derived stem cell (hADSC) | 3.0 × 10⁶ cells | 1.5 × 10⁴ cells |
| Mammary epithelial cell (HMEC) | 3.0 × 10⁶ cells | 1.5 × 10⁴ cells |
| CMF | 12 mg (1.2%) | 0.06 mg (1.2%) |
| Fibrinogen | 6.6 mg | 0.33 mg |

### <Fixation and immunostaining of three-dimensional tissue>

Fixation and immunostaining of the three-dimensional tissue in Example 1 were performed as follows. After culture was performed for 7 days, the three-dimensional tissue in Example 1 was scooped up with a spoon and placed in an Eppendorf tube including 500 µL of PBS. The PBS was removed from the Eppendorf tube and PBS was added again. This was performed three times, and the three-dimensional tissue was washed. After the washing was performed with PBS, 500 µL of 4% paraformaldehyde was added and left to stand overnight. Thereafter, the paraformaldehyde was removed, and the three-dimensional tissue was washed three times with PBS. 100 µL of 0.05% Triton X-100 PBS was placed in each Eppendorf tube and left to stand at room temperature for 15 minutes. The supernatant was removed, and the three-dimensional tissue was washed three times with PBS. Thereafter, 1% BSA-PBS was added, and the three-dimensional tissue was left to stand at room temperature for 60 minutes. As a primary antibody, an anti-CK8/18 antibody or an anti-CK14 antibody was diluted 100-fold with 1% BSA-PBS, and then 100 µL of the diluted solution was placed into each Eppendorf tube and left to stand overnight at 4°C. The three-dimensional tissue was washed three times with PBS, and then secondary antibodies (anti-Alexa Fluor (trademark) 546-conjugated goat anti-rabbit IgG secondary antibody or anti-Alexa Fluor (trademark) 647-conjugated goat anti-mouse IgG secondary antibody, diluted 1/200) diluted with 1% BSA-PBS, Hoechst (diluted 1/1000), and Nile Red (diluted 1/1000) were added and left to stand at room temperature for 2 hours. The three-dimensional tissue was washed three times with PBS and then observed using a confocal laser scanning microscope FV3000.

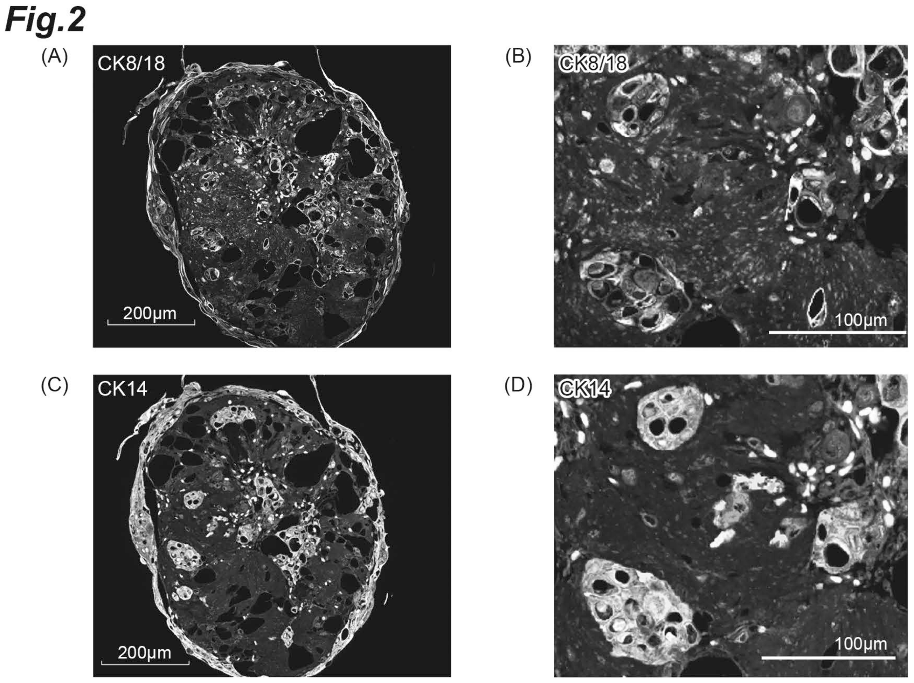

FIG. 2(A) shows observation results of a cross section of the three-dimensional tissue in Example 1 by CK8/18 staining. FIG. 2(C) shows observation results of a cross section of the three-dimensional tissue in Example 1 by CK14 staining. FIG. 2(B) and FIG. 2(D) are enlarged views of portions of FIG. 2(A) and FIG. 2(C), respectively. In the three-dimensional tissue in Example 1, it was observed that CK8/18 and CK14, which are differentiation markers expressed in the mammary gland of a living body, were expressed. In addition, the three-dimensional tissue in Example 1 had a void surrounded by two layers of mammary epithelial cells formed by mammary epithelial cells on the luminal side, which are CK8/18 positive cells, and mammary epithelial cells on the basement membrane side, which are CK14 positive cells. Thus, it was shown that the three-dimensional tissue in Example 1 contains differentiated mammary epithelial cells and is closer to the mammary gland of a living body.

Note that similar results were obtained when a mixed medium of MEGM and a KBM medium suitable for culturing vascular endothelial cells, containing 10 µM insulin, was used.

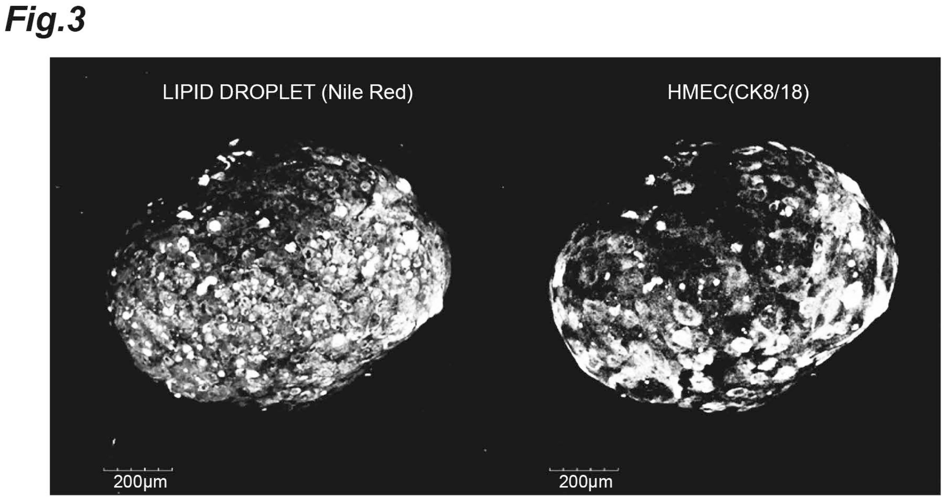

FIG. 3 shows observation results of the three-dimensional tissue in Example 1 using Nile Red staining (left) and CK8/18 staining (right). It was found that lipid droplets had accumulated within the three-dimensional tissue in Example 1 and also within the mammary epithelial cells. This also shows that the mammary epithelial cells in the three-dimensional tissue of Example 1 are differentiated.

Note that similar results were obtained when a three-dimensional tissue was produced by the same method as that for the three-dimensional tissue in Example 1, except that mature adipocytes, adipose-derived stem cells, and mammary epithelial cells were used in a ratio (number of cells) of 1.2:1:0.5.

### [Test Example 2: Comparison of lipid droplets in three-dimensional mammary gland model by Nile Red staining]

### <Production of 3D mammary gland model>

A three-dimensional mammary gland model consisting of a three-dimensional tissue in Example 2 was obtained by a method similar to that for the three-dimensional tissue in Example 1. In addition, a three-dimensional mammary gland model consisting of a three-dimensional tissue in Comparative Example 1 was obtained by a method similar to that for the three-dimensional tissue in Example 1, except that CMF was not used.

### <Fixation and immunostaining of three-dimensional tissue>

In the same manner as in Test Example 1, the three-dimensional tissues in Example 2 and Comparative Example 1 were stained using Nile Red.

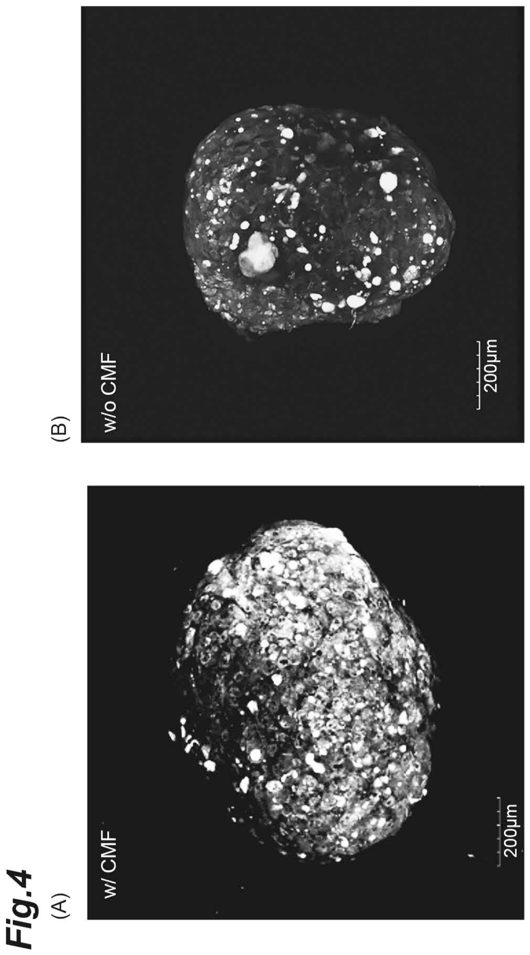

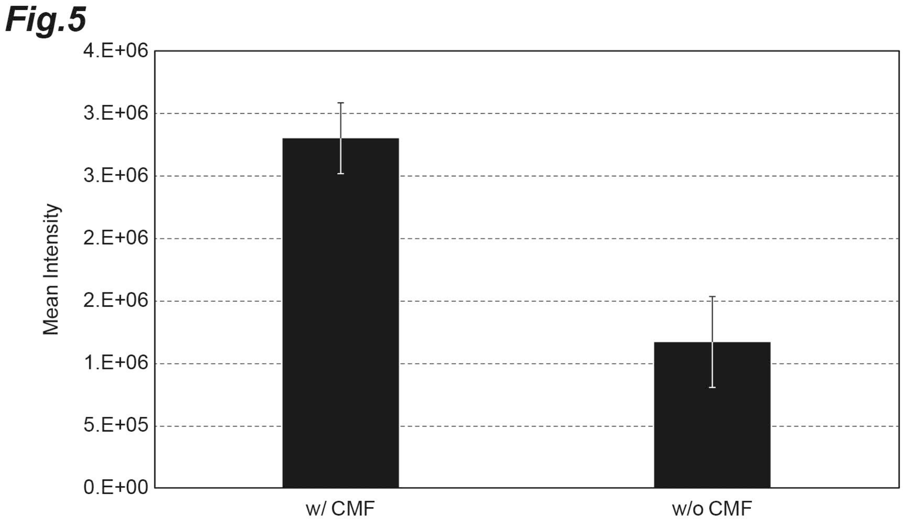

FIG. 4 shows observation results of the three-dimensional tissues in Example 2 and Comparative Example 1 using Nile Red. FIG. 5 is a graph showing the fluorescence intensity of Nile Red in the three-dimensional tissues in Example 2 and Comparative Example 1. The three-dimensional tissue in Example 2 had a higher fluorescence intensity of Nile Red than the three-dimensional tissue in Comparative Example 1. This shows that the three-dimensional tissue in Example 2 can accumulate more lipid droplets than the three-dimensional tissue in Comparative Example 1.

### [Test Example 3: Evaluation of expression level of milk proteins secreted from three-dimensional mammary gland model]

### <Production of three-dimensional mammary gland model>

A three-dimensional mammary gland model consisting of a three-dimensional tissue in Example 3 was obtained by the same method as that for the three-dimensional tissue in Example 1.

### <Quantification of milk proteins>

The three-dimensional tissue in Example 3 was cultured for 7 days in a MEGM medium containing 1 µg/mL prolactin to stimulate milk secretion. Thereafter, casein, which is one of the milk proteins, was detected in the three-dimensional tissue in Example 3. Note that, as a control, casein was also detected in the three-dimensional tissue in Example 3 that had been cultured for 7 days in a MEGM medium not containing prolactin. A similar experiment was also performed on mammary epithelial cells that were seeded at 5×10⁴ cells in a 24-well plate and two-dimensionally cultured for 7 days.

Casein was detected by Western blotting in the three-dimensional tissue in Example 3 or the two-dimensionally cultured mammary epithelial cells by the following procedure. After 7 days of culture in the MEGM medium containing prolactin, proteins were extracted from the three-dimensional tissue in Example 3 or the two-dimensionally cultured mammary epithelial cells using NucleoSpin (registered trademark) RNA/Protein in accordance with a product protocol. An electrophoresis tank and gel were prepared, and the gel was placed in the electrophoresis tank. Then, the electrophoresis tank was filled with 1x SDS buffer, and the inside of the gel was cleaned. Each sample was applied to the gel so that the total amount of proteins contained in the sample was approximately 10 µg, and electrophoresis was performed at 150 to 200 V for approximately 40 minutes. After the electrophoresis ends, the gel was washed with Milli-Q and immersed in 1x transfer buffer for 15 minutes. A membrane was immersed in 100% ethanol for approximately 10 minutes, and the membrane, which had been hydrophilized with ethanol, was placed in the transfer buffer and left to stand for 5 minutes or more. A transfer stack, a membrane, a gel, and a transfer stack were placed in a transfer cassette in this order. The transfer cassette was tilted to remove excess buffer, and then transfer was performed. After the transfer ends, the membrane was washed with 1x TBS-T and blocked with blocking buffer for 1 hour. After the blocking, the membrane was washed with 1x TBS-T and immersed in a primary antibody (anti-casein antibody or anti-α-tubulin antibody) diluted with 1x TBS-T and incubated overnight at 4°C. Then, the membrane was washed with 1x TBS-T and immersed in a secondary antibody (goat anti-rabbit StarBright Blue 700 antibody or Alexa Fluor (trademark) 488-conjugated goat anti-rat IgG secondary antibody) diluted with 1x TBS-T for 1 hour. The membrane was washed with 1 ×TBST, bands were detected using an imager, and the brightness of the bands was quantified using ImageJ.

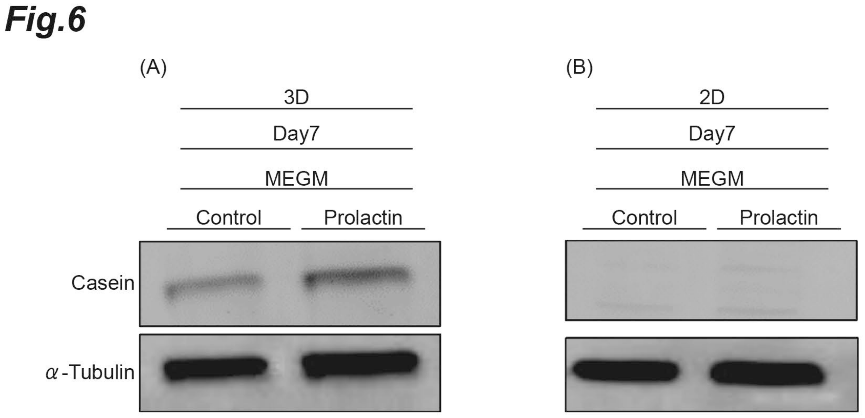

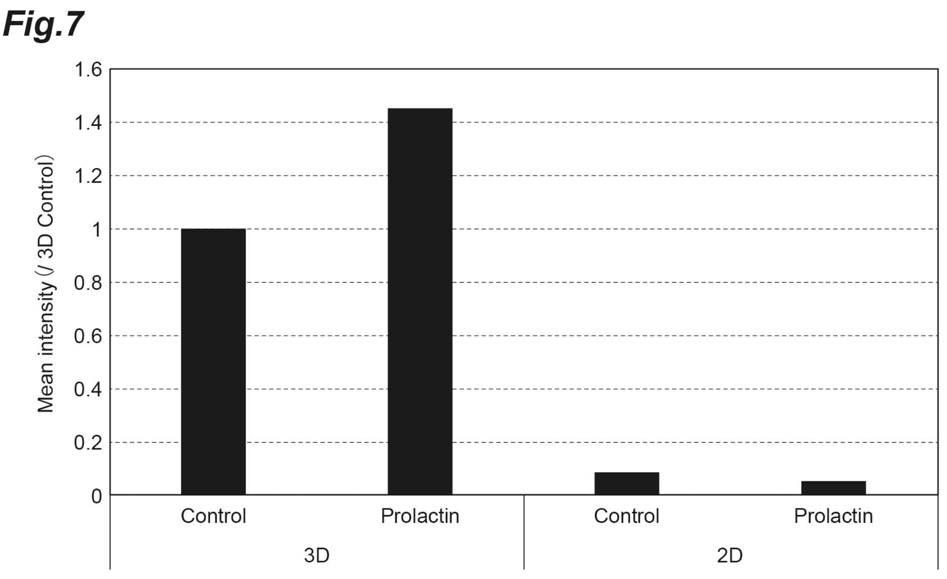

FIG. 6 is an image showing results of detecting casein by Western blotting in the three-dimensional tissue or the two-dimensionally cultured mammary epithelial cells in Example 3. FIG. 7 is a graph showing results of quantifying a band brightness of casein by Western blotting for the three-dimensional tissue or the two-dimensionally cultured mammary epithelial cells in Example 3. Casein could not have been detected in the two-dimensionally cultured mammary epithelial cells, even when milk secretion was stimulated by prolactin. On the other hand, casein was detected in the three-dimensional tissue in Example 3, even when milk secretion was not stimulated by prolactin. Furthermore, when milk secretion was stimulated by prolactin, the amount of casein was increased compared to when milk secretion was not stimulated, indicating that the tissue exhibits responsiveness to substances that stimulate milk secretion.

The above results indicate that milk can be obtained from the three-dimensional mammary gland model in the present invention.

### [Test Example 4: Genetic analysis in three-dimensional mammary gland model]

### <Production of three-dimensional mammary gland model>

A three-dimensional mammary gland model consisting of a three-dimensional tissue in Example 4 was obtained by the same method as that for the three-dimensional tissue in Example 1.

### <Analysis of expression level of tight junction proteins>

The three-dimensional tissue in Example 4 was cultured for 7 days in a MEGM medium containing 1 µg/mL prolactin to stimulate milk secretion. As a control, the three-dimensional tissue in Example 4 was cultured for 7 days in a MEGM medium (not containing prolactin). Thereafter, the expression level of CLDN3, which is one of proteins that configure a tight junction, was analyzed in the three-dimensional tissue in Example 4 by the following procedure. Note that the expression level of CLDN3 was also analyzed in the control three-dimensional tissue.

RNA was extracted from the three-dimensional mammary gland model on the seventh day of culture. NucleoSpin RNA/Protein Kit (model number "740933", manufactured by MACHERY-NAGEL) was used to extract the RNA. The extracted RNA was subjected to a reverse transcription reaction to synthesize complementary DNA (cDNA). QuantiTec (registered trademark) Reverse Transcription Kit (model number "20513", manufactured by QIAGEN) was used to synthesize the cDNA. Quantitative PCR analysis was performed on the synthesized cDNA to analyze the expression level of the CLDN3. Quantitative PCR analysis was performed using Taqman (registered trademark) Fast Advanced Master Mix (model number "4444556", manufactured by Thermo Fisher) and Taqman (registered trademark) Gene Expression assay (model number "4331182", manufactured by Thermo Fisher). Note that, as a primer for quantitative PCR, an oligonucleotide with assay ID: Hs00265816_S 1 was used for the CLDN3. A StepOnePlus real-time PCR system (manufactured by Thermo Fisher) was used as an analysis device, and the analysis was performed in accordance with a thermal profile recommended by the manufacturer.

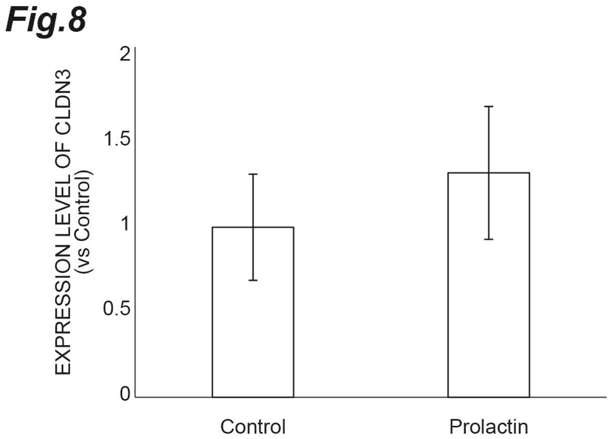

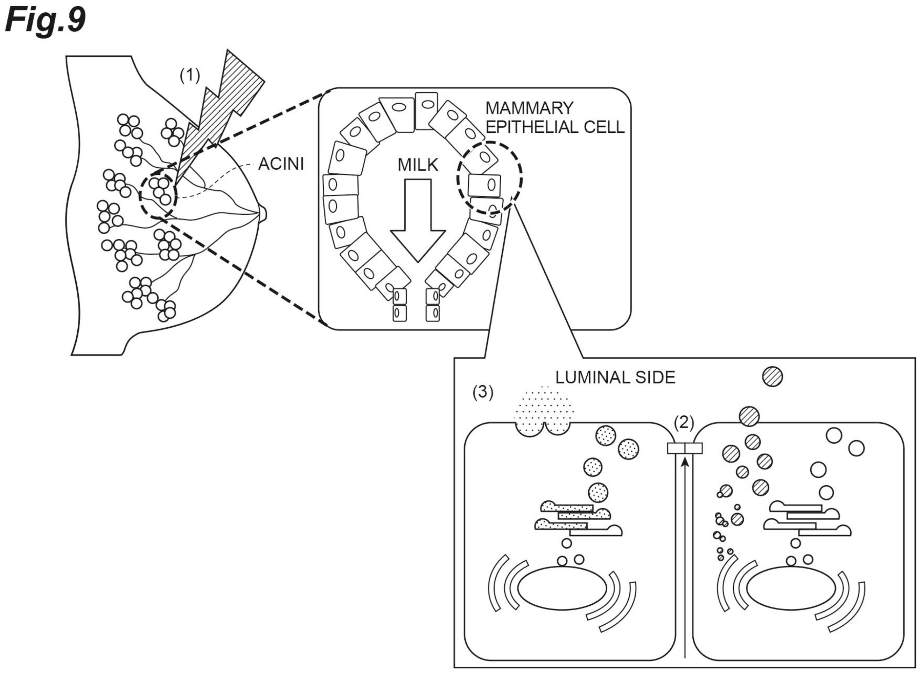

FIG. 8 is a graph showing results of analyzing the expression level of the CLDN3 in the three-dimensional mammary gland model in Example 4. In the three-dimensional mammary gland model in Example 4, a tendency for the expression level of the CLDN3 to increase in response to prolactin was confirmed. As shown in FIG. 9, in a normal milk secretion mechanism in the mammary gland, when the expression level of the CLDN3 increases in response to hormonal stimulation by prolactin ((1) in FIG. 9), tight junctions are formed between mammary epithelial cells in the acini, which is known to prevent the transport of substances between cells ((2) in FIG. 9). After the tight junctions are formed, casein, lactose, milk lipid droplets, and the like are formed and released into the lumen ((3) in FIG. 9). For this reason, in this example, the increase in the expression level of the CLDN3 in response to prolactin was found to suggest the possibility that the three-dimensional mammary gland model may be able to mimic the mechanism of milk secretion in the living body.

### Reference Signs List

1 Mammary epithelial cell on luminal side, 2 Mammary epithelial cell on basement membrane side, 3 Void

## Claims

1. A three-dimensional mammary gland model consisting of a three-dimensional tissue, comprising cells and fragmented extracellular matrix components,
wherein the cells comprise mature adipocytes and mammary epithelial cells.

2. The three-dimensional mammary gland model according to claim 1, having a void surrounded by two layers of mammary epithelial cells.

3. The three-dimensional mammary gland model according to claim 1 or 2, wherein, in the two layers of mammary epithelial cells,
the mammary epithelial cells on a luminal side are CK8/18 positive cells, and the mammary epithelial cells on the basement membrane side are CK14 positive cells.

4. The three-dimensional mammary gland model according to claim 1 or 2, wherein the fragmented extracellular matrix components comprise fragmented collagen.

5. The three-dimensional mammary gland model according to claim 1 or 2, wherein the three-dimensional tissue further comprises a hydrogel.

6. The three-dimensional mammary gland model according to claim 5, wherein the hydrogel is a fibrin gel.

7. A method for producing a three-dimensional mammary gland model, comprising:
a step of bringing cells into contact with fragmented extracellular matrix components in an aqueous medium; and
a step of culturing the cells in contact with the fragmented extracellular matrix components,
wherein the cells comprise mature adipocytes, adipose-derived stem cells, and mammary epithelial cells.

8. The method for producing the three-dimensional mammary gland model according to claim 7, wherein the fragmented extracellular matrix components comprise fragmented collagen.

9. The method for producing the three-dimensional mammary gland model according to claim 7 or 8, wherein
the contact step comprises further bringing a hydrogel precursor into contact, and
the method further comprises a step of gelling the hydrogel precursor after the contact step and before the culturing step.

10. The method for producing the three-dimensional mammary gland model according to claim 9, wherein the hydrogel is a fibrin gel.
